# EUROPEAN PATENT APPLICATION

(11) **EP 2 246 435 A1**
(43) Date of publication of application: **03.11.2010**
(21) Application number: 09159065.3
(22) Date of filing: 29.04.2009
(51) Int. Cl.: C12P 3/00

(54) **Silicon derivate layers/films produced by silicatein-mediated templating and process for making the same**

(71) Applicant: Consiglio Nazionale delle Ricerche - INFM Istituto Nazionale per la Fisica della Materia, 16152 Genova (IT); NanotecMARIN GmbH, 55099 Mainz (DE)
(72) Inventor: Pisignano, Dario, 73100, LECCE (IT); Biasco, Adriana Lucia Angela, 73100, LECCE (IT); Camposeo, Andrea, 73100, LECCE (IT); Pagliara, Stefano, 73100, LECCE (IT); Polini, Alessandro, 73100, LECCE (IT); Mueller, Werner E.G., 65203, WIESBADEN (DE)
(74) Representative: Cattaneo, Elisabetta

(57) **Abstract**

The present invention concerns a process for preparing products having layers/films of silicon derivates comprising the following steps: a) Preparing a mould made of elastomeric material and having a plurality of grooves with mutual spacing in the range from 1 µm to 1mm;b) incubating the mould of step a) in a solution of silicateins in a range of temperatures from 2 to 10°C and in a range of time from few minutes to 10³ hours; c) providing a target substrate of silicon or oxides thereof d) transferring the silicateins from the mould to the said target substrate through soft lithography technique for a time period from few seconds to 10³ hours and removing the elastomeric mould; e) incubating the substrate with patterned silicateins of step d) in a solution of one or more precursors belonging to the class of silane compounds for a time period in a range from few seconds to 10³ hours in a temperature range from 2°C to 25°C. The inventions concerns also a product obtainable by the disclosed process having remarkable electrical features.

## Description

### Field of invention

The present invention concerns layers/films of silicon derivatives obtained by the use of silicatein.

### State of the art

Layers/films of silica, silicates and related materials are widely used in many applications and can be obtained by either conventional processes or biomineralization.

Silica, silicates and related materials (poly-silicic acids, silicic acids, siloxanes, silicones, etc) produced by silicon/oxygen polymerization within amorphous or ordered networks are employed in a variety of scientific, technical laboratory and industrial applications. Layers and films made of these compounds are especially strategic as coatings for biological and biomedical surfaces (for tissue engineering, controlled drug delivery, biocompatible surface modification, transplants, cell adhesion, growth, controlled differentiation, etc), catalytic, diagnostic and sensing surfaces, composites, smart surfaces, optics and optoelectronics (being optically transparent in the visible and in the most of the infrared spectral range, as dielectric layers for interferential elements such as optical filters, mirrors, gratings, distributed Bragg reflectors, distributed feedback laser, for light-emitting devices and displays, photovoltaic cells), for microelectronics and nanoelectronics (as electrically-insulating dielectric layers for field-effect transistors and other electronic devices), as materials for masters and moulds for soft and nanoprint lithographies, etc.

The conventional production of silica surfaces and related materials on laboratory and industrial scale is, however, carried out by processing at high temperatures, pressures and in strongly acid and basic environments (Iler, R. K. in The Chemistry of Silica: Solubility, Polymerization, Colloid and Surface Properties, and Biochemistry, 1979, ed. Iler, R. K. (Wiley, New York), pp. 98-99).

Biomineralization has been regarded as a useful inspirational approach especially in the field of bone-repairing biomaterials: for instance, the application of the siliceous skeleton of sponges as a suitable scaffold onto which human stem cells can be seeded has been recently reported (Green D, Howard D, Yang X, Kelly M, Oreffo RO, Tissue Eng. 2003, 9, 1159).

Although the control over the biomineralization product properties can be accomplished at a variety of levels, including the regulation of particle size, crystal shape/orientation, polymorphic structure, defect texture, and particle assembly, to date, the impact of biomineralization processes on lithographic and technical production processes (for biological, biomedical, sensing, diagnostics, optics, optoelectronics and electronics applications) is still little investigated.

The controlled realization of silica patterns and the production of silica or similar layers by low-cost, gentle biomineralization processes could be instead a feasible and powerful method for manufacturing devices in such fields of application.

Contrary to standard industrial manufacturing, the biological synthesis of silica takes place under mild physiological conditions, at low temperatures and pressures and at near-neutral pH, with clear advantages in terms of cost-effectiveness and environmental impact (Cha JN, Shimizu K, Zhou Y, Christiansen SC, Chmelka BF, Stucky GD, Morse DE, Proc. Natl. Acad. Sci. USA 1999, 96,361). In particular, some peculiar proteins of sponges, called silicateins, catalyze the reaction of silica polymerization to give ordered structures. In such sponges, the siliceous spicules contain a proteinaceous axial filament of silicatein. To date, several isoforms of silicatein have been cloned from the marine sponges and freshwater sponges. These proteins are very similar to cathepsins, a well-known protease family and they are able to coordinate the deposition of silica. At neutral pH, the silicatein filaments and their constituent subunits catalyze the "in vitro" polymerization of silica and silsesquioxanes from tetraethoxysilane and organically modified silicon triethoxides, respectively. Besides this catalytic activity, when the proteins are assembled into mesoscopic filaments (of diameter in the µm range and up to a few millimetres in length), they serve as scaffolds that spatially direct the synthesis of polysiloxanes over the surface of the protein filaments. Hence, these biomolecules present the combined characteristics of chemical action (catalysis) for the formation of silica and patterning action, by driving the silica on the surface of the filaments.

The most abundant silicatein subunit in marine sponges (silicatein-α) was found to be very similar to the protein cathepsin L, that exhibits specific cysteine residues forming intramolecular disulfides. Site directed mutagenesis experiments also have shown that the specific serine (Ser26) and histidine (His165) residues are crucially involved in the catalysis of silica polymerization by silicon alkoxide substrates, and the first silicatein-based biocatalysis process was patented in 2001 by Morse and coworkers (Morse DE, Stucky GD, Deming TD, Cha J, Shimizu K, Zhou Y, Methods, compositions, and biomimetic catalysts for in vitro synthesis of silica, polysilsequioxane, polysiloxane, and polymetallo-oxanes. 2001, United States Patent 6670438). Almost concomitantly, a specific modification of the expression conditions for the production of recombinant silicateins was found and patented by Müller and co-workers (Müller, WEG, Schröder, HC, Lorenz, B, Krasko, A. Silicatein-mediated synthesis of amorphous silicates and siloxanes and use thereof. 2001, United States Patent 7169589). This allows high yields of highly-active proteins to be obtained, and overcomes the drawbacks of time-consuming, laborious and low-throughput achievement of silicateins by both isolation from natural sources and previous state-of-the-art recombinant methods. Importantly, the so-obtained recombinant silicatein exhibits high enzymatic activity, together with a higher pH (4.5-10) and thermal stability with respect to the natural silicatein.

Therefore, some living organisms such as marine and freshwater sponges, such as *Tethya Aurantia* and *Suberites Domuncula,* and diatoms are able to synthesize silica and related materials at room temperature by means of environmental friendly processes catalyzed by biological molecules, thus producing enormous quantities of siloxanes (estimated about gigatons per year).

In general, proteins comprise one or more chains of amino acids, linked by peptide bonds and folded into a specific three-dimensional configuration (tertiary structure). A protein is biologically functional if it is able to fold into its native state rapidly and reliably.

In particular, the catalytic activity of silicatein-α was found to be strictly dependent on its native three-dimensional conformation.

The silicatein-α activity is irreversibly degraded by thermal treatments on the proteins, and it can significantly reduce upon contact with solid surfaces.

To date, the evaluation of the possible structural changes induced by surface adsorption on proteins has not yet been fully addressed. In particular, drying processes often needed by solid-state patterning procedures, together with the complex electrostatic, steric, and hydrophilic/hydrophobic interaction with surfaces may have a negative impact on the protein stability, possibly leading to reversible or irreversible aggregation and loss of functionality.

This makes difficult the practical utilization of silicatein-α for modifying surfaces and for chemically and spatially directing the polymerization of silica and related materials on surfaces, to form layers and films of oxides of technical use. As alternative route, a lot of efforts have been directed to the design and employment of other bio-inspired catalysts which, similarly to silicatein-α, have a nucleophilic functionality and a hydrogen-bonding acceptor group, thus working as enzymes by a mechanism functionally related to that of the silicateins.

To date, however, there is no established method for achieving technically employable films or layers of silica and related materials on surfaces by highly-active recombinant silicatein-α. Some specific mutations in this kind of proteins have been already identified, which are able to optimize the production from the donor organisms, the stability and the catalytic activity. However, also genetically modified and engineered silicateins show inhibition and loss of functionality when placed into contact with solid surfaces. This limit makes difficult their exploitation in some applications, for example the production of layers and derivatives by means of biomineralization processes.

The document EP1905869 describes a process for the formation of monolayer or multilayer film of inorganic material obtained by means of inorganic material-binding peptides, which, deposited on the solid surface as monolayer of aggregates, catalyze the biomineralization reaction following to the contact with different precursors or substrates. Among the various alternatives for the realization of the peptide capable to bind the inorganic material, the authors propose the use of a portion of silicatein extracted from diatomes for the realization of monolayers and multilayers of TiO₂ and monolayers of SiO₂.

The document US20030003223 describes a process for manufacturing substrates functionalized to bind proteins containing histidines. In this way the proteins can be deposited on the substrates according definite patterns, while maintaining their own enzymatic activity. Among the possible proteins proposed to bind the substrates, silicateins are cited in order to obtain silica and derivatives on the surface of the substrate, by exploiting the bio-mineralization process. The process illustrated in the document US20030003223 provides for the linker to bind the proteins to the substrate.

The document W02008022774 proposes a process for the use of recombinant proteins containing portions of silicateins for the synthesis of silica, siloxanes and derivatives and their use in dental field. Particularly, a recombinant protein, containing an "adhesive" portion in order to facilitate the adhesion to the teeth surface and other solid materials and another portion of silicatein leading the catalytic activity, is claimed. W02008022774 is focused on the engineering and optimization of the recombinant protein for applications in dental field.

An object of the present invention is therefore to provide layers/films of silica and derivates, having desired structure and thickness on suitable supports, by using silicatein, thus allowing to avoid the use of a linker in order to bind the desired substrate.

A further object is to provide layers/films of silica and derivates, that are insulating and optically transparent layers/films on suitable substrates.

### Summary of the invention

These objects have been achieved by the process for making layers based on silicon by means of highly-expressed and highly-active silicateins, by using bio-mineralization processes.

The invention therefore relates to a process for preparing products having layers/films of silicon derivates comprising the following steps:
a) Preparing a mould made of elastomeric material and having a plurality of grooves with mutual spacing in the range from 1 µm to 1 mm;
b) incubating the mould of step a) in a solution of silicateins in a range of temperatures from 2 to 10°C and in a range of time from few minutes to 10³ hours
c) providing a target substrate of silicon or oxides thereof;
d) transferring the silicateins from the mould to the said target substrate through soft lithography technique for a time period from few seconds to 10³ hours and removing the elastomeric mould;
e) incubating the substrate with patterned silicateins of step d) in a solution of one or more precursors belonging to the class of silane compounds for a time period in a range from few seconds to 10³ hours in a temperature range from 2°C to 25°C.

According to the invention a continuous layer of silicon derivates, such as silica, silicates, siloxanes or related materials of good quality, has been obtained, which can be subsequently used for the desired uses such as dielectric layer for organic thin film transistors.

In the present invention, when the term:
- "silicatein" is used, it is intended to comprise recombinant silicateins, silicateins isolated from natural sources after gene induction, as well as silicatein-fusion proteins;
- "silicon derivates" is used, it is intended to refer to all the silicon compounds with oxygen, such as silica, silicon oxides, silicates, siloxanes, etc.

The invention relates also to a product comprising a substrate of silicon or oxides thereof and layers/films of silicon derivatives obtainable by the process of the invention and the technical use thereof in biological, biomedical, sensing, diagnostic, optical, catalytic, electronic and optoelectronic applications.

The product of the invention shows the surprising and peculiar technical feature of a continuous layer of silicon derivates on the target substrate. Furthermore, such a product has remarkable and surprising electrical characteristics. Measured leakage currents through produced layers are below 10 nA under bias voltage up to 10 V.

The layers obtained by applying the process of the invention turned out to have optimal chemical-physical characteristics and were used for the realization of devices such as organic thin film transistors having functionality higher than those realized by applying other known bio-mineralization techniques. A particularly preferred embodiment of the present invention relates to electrically insulating and optically transparent silicon layers produced by templating of recombinant silicateins.

### Description of the figures

The invention will be now described with reference to the annexed figures, wherein:
- Figure 1 is the scheme of incubation step e) with a precursor of the example of the invention;
- Figure 2 reports the biosilicification reaction progress investigation using atomic force microscope;
- Figure 3a is the SEM micrograph of grown biosilica after 120 hours of TEOS incubation;
- Figure 3b is EDX spectra of bare silicon (open circles), silica layers with partial oxide formation (red circles) and complete silica layers afters 120 hours of TEOS incubation (continuous black line); energy beam = 10 keV;
- Figure 4 is two-terminal electrical characterizations on biosilica layer samples, produced by 120 and 132 hours incubation in a TEOS buffer solution; leakage current was measured to be < 10 nA through the biosilica layers when the voltage bias was increased up to 10 V;
- Figure 5 shows the reproducibility of the two-terminal electrical measurements performed on a same biosilica layer in the example of the use of the product of the invention;
- Figure 6 shows two-terminals electrical measurement on the negative control (sample after 120 hours incubation in a TEOS buffer solution without recombinant silicatein deposited), which shows similar electrical characteristics as Au/Si contact.

### Detailed description of the invention

The invention concerns a process for preparing products having layers/films of silicon derivates comprising the following steps:
a) Preparing a mould made of elastomeric material and having a plurality of grooves with mutual spacing in the range from 1 µm to 1 mm;
b) incubating the mould of step a) in a solution of silicateins in a range of temperatures from 2 to 10°C and in a range of time from few minutes to 10³ hours
c) providing a target substrate of silicon or oxides thereof;
d) transferring the silicateins from the mould to the said target substrate through soft lithography technique for a time period from few seconds to 10³ hours and removing the elastomeric mould;
e) incubating the substrate with patterned silicateins of step d) in a solution of one or more precursors belonging to the class of silane compounds for a time period in a range from few seconds to 10³ hours in a temperature range from 2°C to 25°C.

According to the invention step a) of the process is the preparation of a mould of elastomeric material having a plurality of grooves with mutual spacing in the range from 1 µm to 1 mm. Preferably the plurality of grooves are parallel channels. Such grooves can be of any kind of form, i.e. of any pattern, such as rectangules, squares, lines of any curvature, fractal graphs, (being the list not limitative in relation to the scope of the invention), being grooves mutually spaced each other by 1 µm to 1 mm. Such plurality of grooves has a mutual spacing, preferably, in the range from 1 µm to 50 µm, more preferably of at least 30 µm.

Preferably, such a preparation is carried out by replica moulding from a master. The elastomeric material is preferably PDMS (polydimethylsiloxane), more preferably polydimethylsiloxane having high young module. As elastomeric material fluoroelastomers can also be used. The first step of the process concerns the realization of elastomeric replicas of an existing, lithographically-made master micro or nanostructures, by conventional replica moulding (REM) methods. In the REM recipe, polydimethylsiloxane (PDMS, such as the Sylgard™ 184 from Dow Corning) or other elastomers (such as polydimethylsiloxane having high Young module or fluoroelastomers) are employed for realizing elastomeric replicas of masters.

Without wishing to be bound by any theory, the inventors have surprisingly seen that optimal results were obtained with moulds of such geometries, that resulted to promote the deposition of silicateins on solid surfaces and to increase the quality of the layer obtained by mineralization. As a matter of fact, the inventors of the present invention have noticed that the presence of grooves, preferably as parallel channels, on the replica surface greatly promotes the deposition of silicatein protein. In the method of the invention, therefore, the silicatein is patterned on the final target surface with typical features of the grooves, in case of parallel channels having lateral size ranging from few µm to 10 mm. The method includes an optimised scheme for the realization of silicatein patterns immobilized by soft lithography (REM plus micro- or nano-contact printing) technologies on surfaces.

Advantageously, the mould of step a), before being incubated in step b) can be optionally treated by oxygen plasma in order to increase the hydrophilicity and to promote the adsorption of silicateins. After the removal, the moulds are temporarily activated by oxygen plasma under various conditions of process to increase the hydrophilicity of PDMS for the subsequent adsorption of silicatein molecules. According to the invention, best patterning results were achieved by employing an RF power of 50 W, and a plasma duration of 5 seconds.

The step b) of incubating takes place in a solution of silicateins in a range of temperatures from 2 to 10°C and in a range of time from few seconds to 10³ hours. Preferably such a temperature is about 4°C. In a preferred embodiment, the period of time is in the range from 1 minute to 48 hours, more preferably it is about two hours. At the end of the incubation step advantageously a drying substep of the mould is carried out in order to remove the excess solution.

In step c) the target substrate of silicon or oxides thereof is provided. The target substrate can be preferably a substrate of silicon.

The target substrate can advantageously be subjected to a cleaning substep of the surface to remove the native oxide and organic contaminants in order to obtain superficial reactive groups for next immobilization of silicateins.

In a particularly preferred embodiment of the process, the target substrate used for soft lithography is, n-type (Sb-doped) silicon (100), exhibiting a resistivity in the range of 0.005-0.020 Ω cm, a substrate thickness between 325 µm and 375 µm and a surface roughness < 1 nm. Advantageously and preferably, native oxide silicon (100) n-type substrates are cleaved into ∼1 cm² chips by means of a diamond-tipped stylus and cleaned from dust residues with N₂ flux. According to the invention, the cleaning substep of the target surfaces to remove organic contaminations and to generate reactive surface groups, is advantageous for the subsequent immobilization of proteins. The native silicon oxide, naturally produced by air exposure, must be removed from the substrates to have access to the underlying Si surface by means of this facultative cleaning substep. To this aim, silicon (100) n-type substrates are etched by a water solution of concentrated hydrofluoric acid (HF 48%) and a buffering salt, NH₄F, for five minutes. The buffering agent is added to maintain a constant pH as the HF is consumed in its reaction with SiO₂: NH₄F /HF / H₂O (11.3 g/2.8 ml/17 ml). Distilled deionised water with a conductivity of ∼ 1 µS is preferably used.

Step d) consists in transferring the silicateins from the mould to the target substrate through soft lithography technique. Preferably step d) is carried out by means of conformal contact printing. Such step occurs for a time period from few seconds to 10³ hours, preferably in the range from 1 minute to 10 hours, more preferably within about two hours, before removing the elastomeric mould. Conformal contact printing resulted to be a simple, fast, aspecific immobilization method, namely physisorption, in order to transfer silicateins from the elastomeric elements to the target surface. Aspecific immobilization typically relies on interactions between the surface and amino-acidic side groups of proteins. These interactions are mainly hydrophobic, dipolar or electrostatic. In this process, proteins generally adsorb on a surface in a non specific and random way. According to the invention, physisorption methods exhibited superior features in terms of ease of operation, pattern definition, and reproducibility.

Step e) consists in incubating the target substrate with patterned silicateins of step d) in a solution of one or more precursors belonging to the class of silane compounds for a time period in a range from few seconds to 10³ hours in a temperature range from 2°C to 25°. Preferably step e) is carried out for a time period in the range from 1 to 200 hours, more preferably of at least 120 hours at a temperature of 4°C. More preferably, such a precursor is tetraethyl orthosilicate (TEOS).

With respect to the prior art, the present process allows the following advantages to be obtained:
- low process temperatures (4°C),
- direct patterning of the silicateins on the target substrate (without using linkers),
- formation of high quality layers, obtained through bio-mineralization, and
- production of devices of high functionality by using layers obtained by applying the proposed process.

A particularly preferred embodiment relates to silica layers produced through recombinant silicatein templating.

According to the invention, a continuous layer of silicon derivates was obtained. Preferably, such a layer is a continuous and homogeneous silica layer after incubation with organic precursors with insulating electrical features.

### Example.

### Preparation of the product of the invention

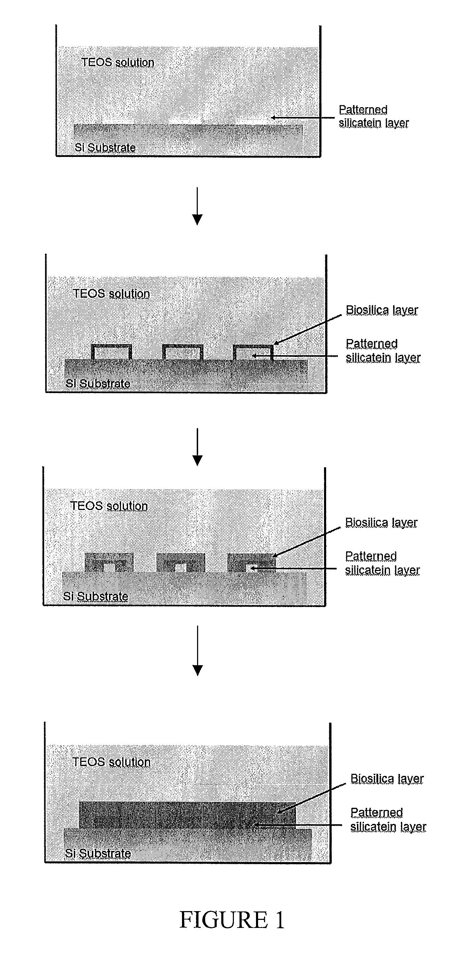

The employed recombinant silicatein protein (molecular mass = 25041.5 Dalton; pl = 6.2) was diluted in a buffer containing 6 M urea, 300 mM KCI, 50 mM KH₂PO₄,250 mM imidazole, at pH 8. This buffer was preferable for stabilizing the enzyme. After plasma treatment, the elastomeric moulds were immediately inked with the recombinant silicatein protein solution (50 µg/ml) and left in incubation at a temperature of 4°C for about 2 hours, thus allowing molecules to adsorb onto the PDMS patterned surface. The mould was then dried by removing the excess solution with a micropipette and left under laminar air flow for 20 minutes. Afterwards, it was gently pressed onto the surface to be patterned and left in contact for 2 or more hours. During the conformal contact printing, molecules were transferred from the mould to the surface according to the well-established REM printing. Afterwards, the elastomeric replica was removed and the silicon substrate with patterned silicateins was incubated in a precursor solution, i.e. 99% TEOS at 4°C for at least 120 hrs. The treatment step with a precursor was a crucial step of the process of the invention and it is depicted in Figure1. Upon TEOS incubation, the original protein pattern, which was made of parallel separated features to facilitate the REM procedure, templated the production and accumulation of silica according to known biomineralization mechanisms. Consequently, the gradual growth of the biosilica produced first an increase of the pattern duty cycle with respect to the original protein pattern, and finally the formation of a continuous oxide layer, ready to be employed for technical uses.

### Characterization of the obtained product:

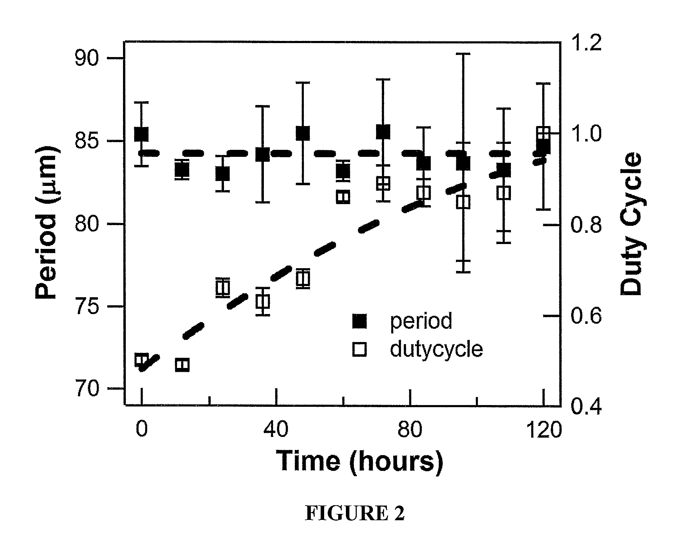

The silicatein patterns and silica layers obtained in the above example were investigated by using fluorescence microscopy. A protocol normally used to dye proteins in solution was applied to protein patterns on surface. As fluorophore, fluorescein isothiocyanate (FITC), in dimethyl sulfoxide (DMSO) buffer, was used. FITC was the original fluorescein molecule functionalized with an isothiocyanate group (-N=C=S), by replacing a hydrogen atom on the bottom ring of the structure. This derivative was reactive towards amine groups on proteins. The scheme was as follows: 3.5 ml of buffer bicarbonate 0.1 M (NaHC0₃ solution A) were mixed with 315 µl of Buffer Carbonate 0.1 M (Na₂COₛ solution B). The substrate was incubated in 1300 µl A + B, to which 100 µl FITC/DMSO solution (1.5 mg in 1 ml DMSO) were added. Samples were incubated at 4°C for 8 hours under shaking conditions. Afterwards, they were washed 3 times with PBS (5 minutes each) and immediately observed. Protein patterns were also investigated by rhodamine B isothiocyanate (0.1 mg/ml in methanol). Samples were incubated at 4°C for 8 hours under shaking conditions, washed 3 times with PBS (5 minutes each) and immediately observed. In both cases isothiocyanate reacted with amine groups forming thiourea. These fluorescence methods, though not specific for silicatein, allowed image patterned areas to be easily and quickly formed. During incubation with the silica precursor (scheme in Figure 1), the biosilicification reaction was stopped every 12 hrs and samples were investigated by fluorescence microscopy using rhodamine 123. Solutions of 0.1 mg/mL for rhodamine 123 were made in methanol. Samples were incubated at 4°C for 8 hours under shaking conditions. Afterwards, they were washed 3 times with PBS (5 minutes each) and immediately observed. Green fluorescing silica stripes of size around 40 µm were routinely observed. In the absence of TEOS precursors, no pattern was observable (negative control of the process). The residual rhodamine staining in this case was likely due to un-removed PDMS fragments. After 24 hours of TEOS incubation, the silica growth occurs only on microprinted areas, and empty stripes were still visible between patterned protein features. As the incubation time increased, the area was covered with silica increased too, the original protein pattern being initially still appreciable under the biomineralized layer. For incubation times longer than 120 hours, the resulting silica layer covered the underlying silicatein pattern, thus resulting in a continuous film. The biosilicification reaction progress was also investigated using atomic force microscope (AFM). In this case, the stripes width was measured by comparing topography and phase imaging at each precursor incubation time as reported in Figure 2. Specifically, in Figure 2 silica pattern period (full squares) and duty cycle (stripe width/period, open squares) measured by AFM and optical microscopy for various TEOS incubation time. The pattern period was almost constant around 85 µm, whereas the duty cycle increased from 0.49 to 1 (silica coverage). Dotted lines were guides for the eye only.

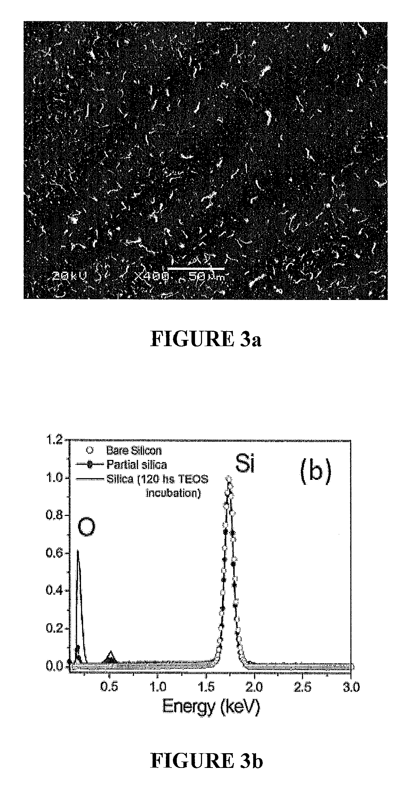

The biosilicification reaction progress was also investigated using scanning electron microscopy and energy dispersive x-ray analysis. A SEM micrograph of a produced silica layer (after 120 hours of TEOS incubation) is shown in Figure 3a, confirming the AFM finding of a continuous oxide growth. The EDX analysis of the produced layers (at 10 keV beam energy corresponding to a penetration depth in the sample of 1-2 µm) evidenced the enhancing of the oxygen peak upon biosilification, thus confirming the silica-like chemical composition of the resulting films (Figure 3b).

### Use of the product of the invention

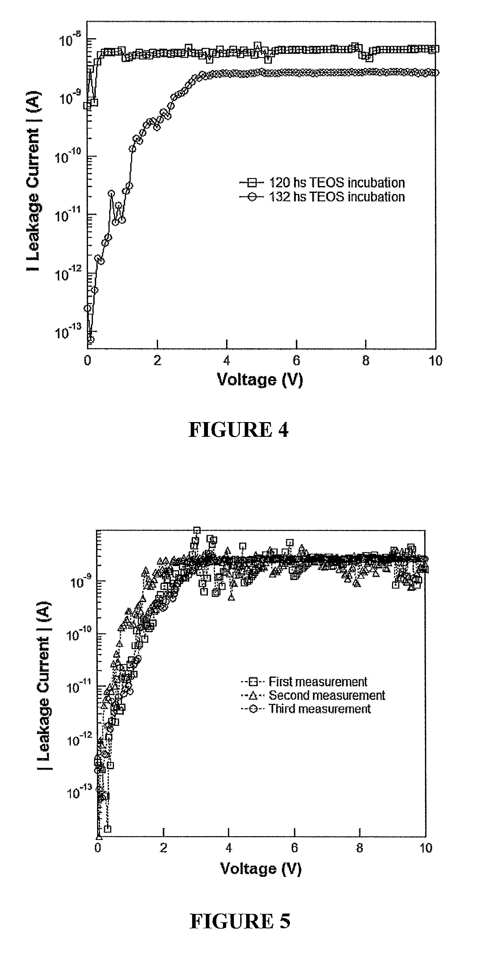

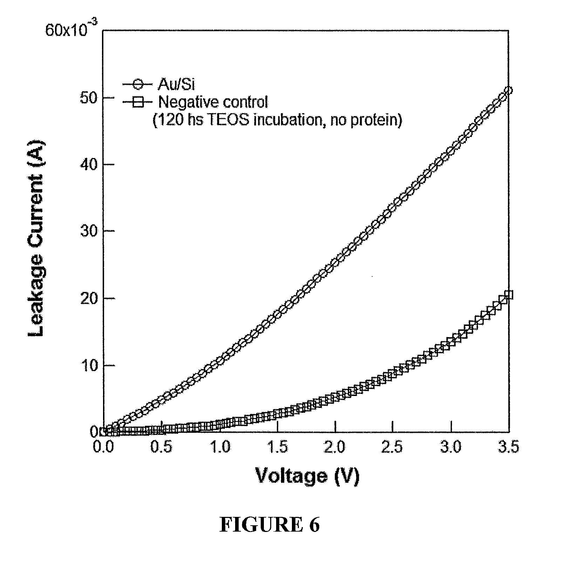

A preferred technical use of the resulting silica layers is the use as a possible dielectric layer for electronic or optoelectronic devices, such as the gate insulator in an organic thin film transistor (OTFT). Briefly, a stripe of biosilica (width = 300 µm, length = 300 µm) was grown by biosilicification process on heavily n-doped (As, resistivity < 0.006 Ωcm) Silicon. The use of Si/Si0₂ wafers (thickness of the commercial silica = 100 nm), with exposed Si areas by conventional wet etching (HF:NH₄F:H₂O, 7:37:56 in weight), was also tested. A thin film (100 nm) of Au was thermally evaporated through a metal shadow mask in a region (2x2 mm²) across biosilica and commercial silica surfaces. Two-terminal electrical characterizations were performed by contacting the thin film of gold on the commercial silica and back-contacting heavily n-doped silicon with two 20-µm diameter tungsten probe-tips, positioned with micrometer accuracy through two manual probe-heads. Electrical signals were analyzed by a Keithley 4200 Semiconductor Characterization System (resolution down to 10 fA for detected current < 10 nA). Under small bias voltage (1 V), leakage currents in the range 1 - 10³ pA were measured through the biosilica layer for samples realized with an incubation time in a TEOS buffer of less than 120 hours, probably due to the presence of defects and local unhomogeneities in the biosilica layer. On the contrary, samples produced by a TEOS incubation of 120 and 132 hours exhibited good insulating behaviour (leakage currents < 10 nA) under bias voltage up to 10 V (as represented in Figure 4). Several measurements performed on the same device produced similar electrical characteristics, thus showing the reproducibility of the measurement and the stability of the device under test (as shown in Figure 5). As a negative control, the same measurements were performed on samples after 132 hours incubation in the same TEOS buffer solution, but without silicatein deposited on the investigated surface by the above described method. Measurements performed on different samples showed large leakage currents (20 mA) even at small bias voltage (3 V). Such currents were close to those measured by contacting Au thin films directly deposited on heavily n-doped silicon, thus indicating no electrically insulating properties as shown in Figure 6.

## Claims

1. A process for preparing products having layers/films of silicon derivates comprising the following steps:
a) Preparing a mould made of elastomeric material and having a plurality of grooves with mutual spacing in the range from 1 µm to 1 mm;
b) incubating the mould of step a) in a solution of silicateins in a range of temperatures from 2 to 10°C and in a range of time from few minutes to 10³ hours;
c) providing a target substrate of silicon or oxides thereof;
d) transferring the silicateins from the mould to the said target substrate through soft lithography technique for a time period from few seconds to 10³ hours and removing the elastomeric mould; and
e) incubating the substrate with patterned silicateins of step d) in a solution of one or more precursors belonging to the class of silane compounds for a time period in a range from few seconds to 10³ hours in a temperature range from 2°C to 25°C.

2. The process according to Claim 1, wherein in step a) the grooves of the mould are parallel channels.

3. The process according to Claim 1 or Claim 2, wherein such plurality of grooves has a mutual spacing, preferably, in the range from 1_µm to 50 µm, preferably of at least 30 µm

4. The process according to anyone of Claims from 1 to 3, wherein step a) of the process is carried out by replica moulding from a master.

5. The process according to anyone of Claims from 1 to 4, wherein the elastomeric material of the mould is PDMS (polydimethylsiloxane).

6. The process according to anyone of Claims from 1 to 5, wherein the mould of step a), before being incubated in step b), is treated by oxygen plasma.

7. The process according to Claim 6, wherein an RF power of 50 W, and a plasma duration of 5 seconds was used in the treatment with oxygen plasma.

8. The process according to anyone of Claims from 1 to 7, wherein the incubation step of step b) occurs at 4°C.

9. The process according to anyone of Claims from 1 to 8, wherein the period of time in step b is in the range from 10 minutes to 48 hours, preferably it is about two hours.

10. The process according to anyone of Claims from 1 to 9, wherein at the end of step b) of incubation occurs the drying step in order to remove the excess solution.

11. The process according to anyone of Claims from 1 to 10, wherein in step c), the silicon target substrate is subjected to a cleaning step.

12. The process according to anyone of Claims from 1 to 11, wherein the target substrate used for soft lithography is, n-type (Sb-doped) silicon (100), exhibiting a resistivity in the range of 0.005-0.020 Ω cm, a substrate thickness between 325 µm and 375 µm and a surface roughness < 1 nm.

13. The process according to anyone of Claims from 1 to 12, wherein the soft lithography technique of step d) is conformal contact printing.

14. The process according to anyone of Claims from 1 to 13, wherein step d) is carried out in the range of time from 1 minute to 10 hours, preferably for at least two hours.

15. The process according to anyone of Claims from 1 to 14, wherein in step e) of the process the precursor is tetraethyl ortosilicate (TEOS).

16. The process according to anyone of Claims from 1 to 15, wherein step e) is carried out for a time period in the range from 1 to 200 hours, preferably at least 120 hours at 4°C.

17. The process according to anyone of Claims from 1 to 16, wherein the silicatein solution is a recombinant silicatein solution.

18. A product comprising a substrate of silicon or oxides thereof and layers/films of silicon derivates obtainable by the process according to anyone of claims 1-17.

19. Use of the product according to Claim 18 for the realization of biological and biomedical surfaces, catalytic, diagnostic and sensing surfaces, composites, smart surfaces, dielectric layers for interferential elements such as optical filters, mirrors, gratings, distributed Bragg reflectors, distributed feedback laser, for light-emitting devices and displays, photovoltaic cells, as electrically-insulating dielectric layers for field-effect transistors, as materials for masters and moulds for soft and nanoimprinting lithography.
